# EUROPÄISCHE PATENTSCHRIFT

(11) **EP 2 793 861 B1**
(45) Veröffentlichungstag und Bekanntmachung des Hinweises auf die Patenterteilung: **01.05.2019**
(21) Anmeldenummer: 12801761.3
(22) Anmeldetag: 18.12.2012
(51) Int. Cl.: A61K 9/14, A61K 31/53, A61K 38/15, A61K 47/32

(54) **ZUBEREITUNGEN ENTHALTEND AMORPHES EMODEPSID**
PREPARATIONS CONTAINING AMORPHOUS EMODEPSIDE
PRÉPARATIONS CONTENANT UN EMOPEPSIDE AMORPHE

(30) Priorität: 21.12.2011 EP 11194878
(43) Veröffentlichungstag der Anmeldung: 29.10.2014
(73) Patentinhaber: Bayer Pharma Aktiengesellschaft, 13353 Berlin (DE)
(72) Erfinder: KANIKANTI, Venkata-Rangarao, 51381 Leverkusen (DE); LANGE, Petra, 45219 Essen (DE); HAMANN, Hans-Jürgen, 41539 Dormagen (DE); KLEINEBUDDE, Peter, 40627 Düsseldorf (DE)
(74) Vertreter: BIP Patents
(86) Internationale Anmeldenummer: PCT/EP2012/075909
(87) Internationale Veröffentlichungsnummer: WO 2013/092558

(56) Entgegenhaltungen:
- WO-A1-2010/054833
- DE-A1-102008 022 520
- DE-A1-102009 012 423
- LANGE P: "Emodepsid Bestimmung des Resorptionsortes und Formulierungskonzepte zur Verbesserung der Bioverfügbarkeit", INAUGURAL DISSERTATION HEINRICH HEINE UNIVERSITÄT DÜSSELDORF,, 1. Mai 2011 (2011-05-01), Seiten 1-112, XP007921579,
- BARONSKY J ET AL: "The study of different solid forms of Emodepside", EUROPEAN JOURNAL OF PHARMACEUTICS AND BIOPHARMACEUTICS, ELSEVIER SCIENCE PUBLISHERS B.V., AMSTERDAM, NL, Bd. 71, Nr. 1, 1. Januar 2009 (2009-01-01) , Seiten 88-99, XP025782076, ISSN: 0939-6411, DOI: 10.1016/J.EJPB.2008.06.003 [gefunden am 2008-06-17]

## Beschreibung

Die Erfindung betrifft Zubereitungen enthaltend amorphes Emodepsid in einer Polyvinylpyrrolidon-Matrix, Arzneimittel enthaltend solche Zubereitungen und ihre Verwendung gegen Endoparasiten bei Tieren oder Menschen.

Das anthelmintisch wirksame cyclische Depsipeptid Emodepsid ist aus WO 93/19053 bekannt. Es sind bereits verschiedene Applikationsformen beschrieben worden, so z. B. Extrudate auf Stärkebasis (WO 02/00202), oder als feste Arzneiform mit verzögerter Freisetzung (WO 2009/135593 A2).

Kachi et al (Jpn. J. Pharmacol. 77 (1998) 235-245) beschreibt die amorphen und polymorphen kristallinen Formen des Cycloctadepsipeptids PF1022A.

Schütte (Dissertation, Bonn 2004) beschreibt "Untersuchungen zur Komplexierbarkeit von pharmazeutischen Wirkstoffen mit Amylose durch Extrusion mit Hochamylosestärken". Darin werden auch Emodepsid-Extrudate beschrieben, bei denen Stärke als Basis verwendet wurde.

Emodepsid ist ein schlecht löslicher Arzneistoff mit schlechter Permeabilität. Die Wasserlöslichkeit liegt im Bereich von pH 4 - 10 bei 5 - 7 mg/L.

Solche Wirkstoffe haben oft eine schlechte Bioverfügbarkeit. Es besteht daher Bedarf an Zubereitungen von Emodepsid mit verbesserter Bioverfügbarkeit.

Es wurde nun gefunden, dass amorphes Emodepsid in bestimmten Matrizes eine höhere Wasserlöslichkeit und eine sehr gute Bioverfügbarkeit im Vergleich zum kristallinen Emodepsid zeigt.

Die Erfindung betrifft Zubereitungen enthaltend Emodepsid in amorpher Form in einer Polyvinylpyrrolidon-Matrix.

Die Erfindung betrifft weiterhin Arzneimittel enthaltend solche Zubereitungen.

Die Erfindung betrifft weiterhin die Verwendung der erfindungsgemäßen Zubereitungen oder der Arzneimittel enthaltend die erfindungsgemäßen Zubereitungen zur Bekämpfung von Endoparasiten bei Menschen oder Tieren.

Der INN Emodepside steht für die Verbindung mit dem systematischen Namen: Cyclo[(R)-lactoyl-N-methyl-1-leucyl-(R)-3-(p-morpholinophenyl)lactoyl-N-methyl-1-leucyl-(R)-lactoyl-N-methyl-1-leucyl-(R)-3-(p-morpholinophenyl)lactoyl-N-methyl-1-leucyl. Emodespide ist in WO 93/19053 beschrieben und hat die folgende Formel:

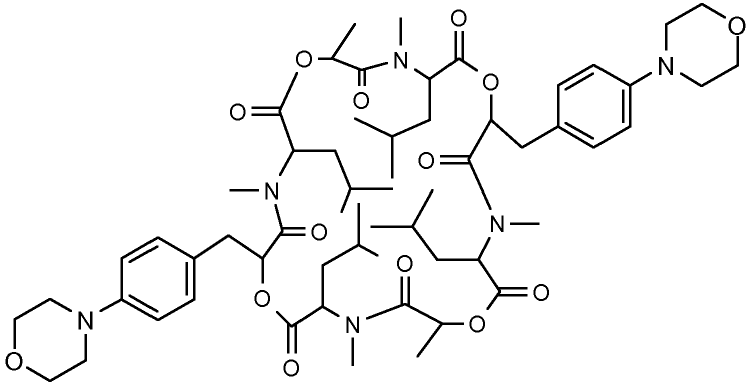

Grundsätzlich können die erfindungsgemäßen Zubereitungen weitere Wirkstoffe enthalten.

Wirkstoffe können je nach Struktur in stereoisomeren Formen oder als Stereoisomerengemische vorliegen, z. B. als Enantiomere oder Racemat. Der Wirkstoff Emodepsid hat insgesamt 8 chirale C Atome - 4 L-Leucin-, 2 D-Milchsäure- und 2 D-Polymilchsäureeinheiten. Allerdings ist die Synthese anenatioselektiv, so dass durch den Fermentationsprozess nur das eine Enantiomer von PF1022A erzeugt wird.

In den erfindungsgemäßen Zubereitungen liegt das Emodepsid im amorphen Zustand vor. Amorph heißt, dass die Atome in einer ungeordneten Struktur vorliegen. Bei einer kristallinen Substanz oder in kristallinen Bereichen haben die Atome sowohl eine Nah-, als auch eine Fernordnung. Amorphes Material hingegen verfügt nur über eine Nahordnung. Der Kristallisierungsgrad des Wirkstoffes kann beispielsweise mit Hilfe der Dynamischen Differenzkalorimetrie oder der Röntgendiffrakotmetrie bestimmt werden.

Bei der kalorimetrischen Messung wird die Schmelzenthalpie, also die notwendige Energie zum Aufschmelzen der Kristalle, gemessen. Liegt der Wirkstoff in einem vollständig amorphen Zustand vor, ist beim Erhitzen keine endotherme Enthalpieänderung messbar.

Bei dem Messverfahren der Röntgenbeugung werden die Abstände zwischen den Molekülketten gemessen. Im amorphen Zustand sind keine regelmäßigen Abstände vorhanden, was zu einer breiten Verteilung und keinen eindeutigen Peaks im Diffraktogramm führt.

Weitere Möglichkeiten zur Überprüfung des amorphen Zustandes sind die Dichtemessung, die Röntgenbeugung, die Infrarot-Spektroskopie und die Kernresonanzspektroskopie.

In den erfindungsgemäßen Zubereitungen liegt ein Anteil von mindestens 50 Gew.-%, bevorzugt mindestens 70 Gew.-%, besonders bevorzugt mindestens 80 Gew.-%, ganz besonders bevorzugt mindestens 90 Gew.-& des Emodepsids in amorphem Zustand vor; wobei die Prozentangaben auf die Gesamtmenge an Emodepsid bezogen sind.

Im Zweifel wird der Gehalt an amorphem Emodepsid durch Dynamische Differenzkalorimetrie bestimmt.

Das Emodepsid liegt in einer Polyvinylpyrrolidon-Matrix vor. Als "Polyvinylpyrrolidon" kommen sowohl reine Polyinylpyrrolidone als auch deren Derivate oder Mischungen von Polyvinylpyrrolidonen und Polyvinylpyrrolidon-Derivaten in Frage.

Polyvinylpyrrolidone (Povidone, PVP) sind im Handel erhältliche hydrophile Polymere. Dabei sind verschiedene PVP-Arten erhältlich. PVP mit niedrigerem Molekulargewicht werden üblicherweise als Bindemittel für Tabletten eingesetzt. Im wässrigen Milieu quellen PVP und erodieren.

Die eingesetzten Polyvinylpyrrolidone oder Polyvinylpyrrolidon-Derivate sind vorzugsweise wasserlöslich. Hierbei handelt es sich in der Regel um lineare nicht quervernetzte Polyvinylpyrrolidone oder Polyvinylpyrrolidon-Derivate.

Die erfindungsgemäße eingesetzten reinen Polyvinylpyrrolidone oder Polyvinylpyrrolidon-Derivate weisen üblicherweise einen K-Wert im Bereich von 12 bis 90, bevorzugt 12 bis 30 auf.

Der K-Wert der Polyvinylpyrrolidone oder Polyvinylpyrrolidon-Derivate steht in Beziehung zur Viskosität und zum Molekulargewicht und kann nach an sich bekannten Methoden bestimmt werden. Im Zweifel werden die Angaben zum K-Wert aus der European Pharmacopeia (Ph. Eur.) verwendet.

Bevorzugt haben die reinen Polyvinylpyrrolidone einen K-Wert von 12 bis 90, besonders bevorzugt von 12 bis 25, ganz besonders bevorzugt von 12 bis 17.

Die Polyvinylpyrrolidon-Derivate sind üblicherweise Polyvinylpyrrolidon-Copolymere. Bei den Polyvinylpyrrolidon-Copolymeren werden vorzugsweise Polymere mit einem K-Wert von 25-30 verwendet.

Ein bevorzugtes Polyvinylpyrrolidon-Derivat ist Copovidon (z. B. Kollidon VA 64 der Fa. BASF). Dabei handelt es sich um ein Copolymer aus Vinylpyrrolidon und Vinylacetat im Verhältnis 6:4 mit einem K-Wert von etwa 30.

Die Zubereitung enthält üblicherweise mindestens 50 Gew.-%, bevorzugt mindestens 66 Gew.-% besonders bevorzugt 75 Gew.-% an Polyvinylpyrrolidon.

Details bezüglich der oben genannten Polyvinylpyrrolidone, Polyvinylpyrrolidon-Derivate und bestimmter Mischungen können dem folgenden Buch entnommen werden: V. Bühler, "Kollidon, Polyvinylpyrrolidone for the pharmaceutical industry,", 9 th revised edition, BASF Pharma Ingredients, Germany, 2008.

Für die Herstellung der erfindungsgemäßen Zubereitungen gibt es mindestens zwei an sich bekannte Methoden: Die Lösungsmittelcopräzipitation und die Schmelzextrusion

Bei der Lösungsmittelcopräzipitation wird Emodepsid zusammen mit dem Polymer im Lösungsmittel gelöst und anschließend das Lösungsmittel beispielsweise unter vermindertem Druck und gegebenenfalls erhöhter Temperatur wieder entfernt. Geeignet sind Lösungsmittel und Lösungsmittelgemische, in denen sich sowohl der Wirkstoff als auch das Polymer lösen. Für die erfindungsgemäßen Zubereitungen sind beispielsweise Ethanol, Acetonitril, Methanol, Aceton und Isopropanol bzw. deren Mischungen geeignet. Als Polymer werden bei der Lösungsmittelcopräzipitation vorzugsweise Polyvinylpyrrolidone mit einem K-Wert zwischen 12-30, bevorzugt 12-17, verwendet, da aus diesen das Lösungsmittel leichter entfernt werden kann als aus Polyvinylpyrrolidonen mit größerem K-Wert,.

Bei der Schmelzextrusion wird der Wirkstoff mit dem Polymer gemischt und in einen Extruder überführt. Die Extrusionstemperatur liegt dabei unterhalb des Schmelzpunktes des Wirkstoffes. Im Fall von Emodepsid kann zwischen 80 und 190°C, bevorzugt zwischen 140 und 180°C extrudiert werden.

Der Schmelzpunkt der thermodynamisch stabilsten Modifikation von Emodepsid liegt bei 192°C. Während der Extrusion löst sich Emodepsid in dem Polymer und präzipitiert beim Abkühlen im amorphen Zustand. Generell werden Polyvinylpyrrolidone mit niedriger Glasübergangstemperatur bevorzugt, um die Stabilität des Wirkstoffs nicht zu gefährden. Darüber hinaus darf sie nicht zu niedrig sein, um eine gewisse Lagerstabilität zu garantieren. Empfehlenswert sind Polymere mit einer Glasübergangstemperatur von mindestens 80 °C aber deutlich unterhalb des Schmelzpunkts von Emodepsid, also 80 °C bis 160 °C, bevorzugt 80°C bis 140 °C. Die Glasübergangstemperatur von Polyvinylpyrrolidon mit einem K-Wert von 12 liegt bei etwa 90 °C und bei einem K-Wert von 25 etwa bei 155°C.

Bei der Schmelzextrusion können dem System zusätzlich Tenside zugesetzt werden. Es kommen grundsätzlich übliche pharmazeutisch annehmbare pulverförmige oder flüssige Tenside in Frage. Als Beispiele seien genannt: Polyoxyethylenglycerolricinoleat 35, Macrogolglycerolhydroxystearat 40, aber auch Gallensalze, Lecithine und nichtionische Tenside wie Natriumdodecylsulphat. Weiterhin seien als Beispiele genannt: Die Polysorbate 20, 60 oder 80 sowie Poloxamere.

Die erfindungsgemäßen Zubereitungen können als solche direkt verwendet werden oder sie werden unter Zugabe weiterer Hilfsstoffe weiterverarbeitet. Dabei liegen sie sowohl für die direkte Anwendung als auch für die Weiterverarbeitung vorzugsweise nach einem Mahlschritt in Form eines Granulats oder Pulvers vor.

"Arzneimittel" im Sinne dieser Erfindung können die Zubereitungen selbst sein oder aber Mittel, die neben den Zubereitungen auch noch pharmazeutisch annehmbare Hilfsstoffe enthalten.

Als orale Arzneimittelformen kommen Pulver, Granulate, Supensionen, Kapseln oder Tabletten in Frage, Tabletten sind bevorzugt.

Als mögliche Hilfsmittel seien genannt: Füllstoffe, Gleitmittel, Schmiermittel, Sprengmittel, Tenside etc..

Als Füllstoffe kommen für feste Zubereitungen (z. B. Tabletten) übliche Füllstoffe, wie zum Beispiel pharmazeutisch verwendete Stärken, z. B. Kartoffel-, Weizen-, Mais- und Reisstärke, verschiedene Mono- und Disaccharide, z. B. Glucose, Laktose und Saccharose, die Zuckeralkohole Mannit und Sorbit in Frage. Auch kolloidale Carbonate wie Calciumcarbonate, Hydrogencarbonate, Kochsalz, Aluminiumoxide, Kieselsäuren, Tonerden und Phosphate (vor allem Calciumphosphate) können zum Einsatz kommen, wobei auch verschiedene Füllstoffe miteinander kombiniert werden können. Als Füllstoffe mit zusätzlichen Trockenbindeeigenschaften werden Cellulosen verwendet, bevorzugt die mikrokristalline Cellulose. Die Gesamtmenge an Füllstoff(en) liegt üblicherweise bei 5-80 % (m/m), bevorzugt 10 bis 70 % (m/m), besonders bevorzugt 20 bis 50 % (m/m).

Weiterhin können die erfindungsgemäßen festen Arzneimittelzubereitungen neben dem oder den Wirkstoffen und anderen vorstehend genannten Inhaltsstoffen noch weitere Hilfsstoffe enthalten. Als Gleitmittel finden z.B. kolloidales Siliciumdioxid, hydrierte pflanzliche Öle, Stearinsäure, Talkum oder deren Mischungen gegebenenfalls in Mengen von üblicherweise 0,1 bis 2 %, bevorzugt 0,5-1,5 % (m/m) Verwendung. Schmiermittel, wie z. B. Magnesiumstearat, sind gegebenenfalls in Mengen von üblicherweise 0,3-2 % (m/m), bevorzugt 0,5 bis 1,5 (m/m) enthalten. Darüber hinaus können Sprengmittel, wie z.B.: Croscarmellose Natrium, in Mengen von üblicherweise 1-10% (m/m) der Rezeptur zugesetzt werden. Aber auch die Verwendung höhere Konzentrationen, wie 10-40 % sind möglich. Tenside, z.B. Natriumdodecylsulphat, üblicherweise 0,1-1 % (m/m), bevorzugt 0,5-1 % (m/m) können zur besseren Benetzung hinzugefügt werden. Weitere Tenside, die eingearbeitet werden können sind die nichtionogenen Tenside Polyoxyethylenglycerolricinoleat 35, Macrogolglycerolhydroxystearat 40, polyoxyliertes Sorbitan-Monooleat, Sorbitan-Monostearat, Ethylalkohol, Glycerinmonostearat, Polyoxyethylstearat und Alkylphenolpropylglykolether, die ampholytische Di-Natrium-N-lauryl-ß-iminodipropionat und Lecithin und die anionenaktive Natriumlaurylsulphat, Fettalkoholethersulphat und Mono-/Dialkylpolyglykoletherorthophosphorsäureester-Monoethanolaminsalze.

Zur Verbesserung der Palatabilität können des weiteren Aromen und/oder Geschmacksstoffe der Rezeptur hinzugefügt werden.

Die erfindungsmäßigen Zubereitungen können z. B. hergestellt werden, indem man die Bestandteile mischt oder granuliert und dann zu Tabletten verpresst. Bevorzugt ist dabei eine Direkttablettierung der Einsatzstoffe, das heißt, dass alle Einsatzstoffe gemischt werden und das aus der Mischung ohne weitere Arbeitsschritte wie Granulieren, o. ä. direkt Tabletten verpresst werden.

Die erfindungsgemäßen Zubereitungen bzw. die erfindungsgemäßen Arzneimittel eignen sich bei günstiger Warmblütertoxizität zur Bekämpfung von pathogenen Endoparasiten, die bei Menschen und in der Tierhaltung und Tierzucht bei Nutz-, Zucht-, Zoo-, Labor-, Versuchs- und Hobbytieren vorkommen. Sie können gegen alle oder einzelne Entwicklungsstadien der Schädlinge sowie gegen resistente und normal sensible Endoparasiten-Isolate eingesetzt werden. Durch die Bekämpfung der pathogenen Endoparasiten sollen Krankheit, Todesfälle und Leistungsminderungen (z. B. bei der Produktion von Fleisch, Milch, Wolle, Häuten, Eiern, Honig usw.) vermindert werden, so dass durch den Einsatz der Wirkstoffe eine wirtschaftlichere, einfachere und gesündere Tierhaltung möglich ist. Zu den pathogenen Endoparasiten zählen Helminthen wie Platyhelmintha (insbesondere Monogenea, Cestoden und Trematoden), Nematoden, Pentastoma und Acanthocephala. Als Beispiele seien genannt:
**Monogenea:** z.B.: Gyrodactylus spp., Dactylogyrus spp., Polystoma spp..

**Cestoden:** Aus der Ordnung der Pseudophyllidea z. B.: Diphyllobothrium spp., Spirometra spp., Schistocephalus spp., Ligula spp., Bothridium spp., Diphlogonoporus spp..

Aus der Ordnung der Cyclophyllida z. B.: Mesocestoides spp., Anoplocephala spp., Paranoplocephala spp., Moniezia spp., Thysanosoma spp., Thysaniezia spp., Avitellina spp., Stilesia spp., Cittotaenia spp., Andyra spp., Bertiella spp., Taenia spp., Echinococcus spp., Hydatigera spp., Davainea spp., Raillietina spp., Hymenolepis spp., Echinolepis spp., Echinocotyle spp., Diorchis spp., Dipylidium spp., Joyeuxiella spp., Diplopylidium spp..

**Trematoden:** Aus der Klasse der Digenea z. B.: Diplostomum spp., Posthodiplostomum spp., Schistosoma spp., Trichobilharzia spp., Ornithobilharzia spp., Austrobilharzia spp., Gigantobilharzia spp., Leucochloridium spp., Brachylaima spp., Echinostoma spp., Echinoparyphium spp., Echinochasmus spp., Hypoderaeum spp., Fasciola spp., Fasciolides spp., Fasciolopsis spp., Cyclocoelum spp., Typhlocoelum spp., Paramphistomum spp., Calicophoron spp-, Cotylophoron spp., Gigantocotyle spp., Fischoederius spp., Gastrothylacus spp., Notocotylus spp., Catatropis spp., Plagiorchis spp., Prosthogonimus spp., Dicrocoelium spp., Eurytrema spp., Troglotrema spp., Paragonimus spp., Collyriclum spp., Nanophyetus spp., Opisthorchis spp., Clonorchis spp. Metorchis spp., Heterophyes spp., Metagonimus spp..

**Nematoden:** Aus der Ordnung der Trichinellida z. B.: Trichuris spp., Capillaria spp., Trichomosoides spp., Trichinella spp..

Aus der Ordnung der Tylenchida z. B.: Micronema spp., Strongyloides spp..

Aus der Ordnung der Rhabditina z. B.: Strongylus spp., Triodontophorus spp., Oesophagodontus spp., Trichonema spp., Gyalocephalus spp., Cylindropharynx spp., Poteriostomum spp., Cyclococercus spp., Cylicostephanus spp., Oesophagostomum spp., Chabertia spp., Stephanurus spp., Ancylostoma spp., Uncinaria spp., Bunostomum spp., Globocephalus spp., Syngamus spp., Cyathostoma spp., Metastrongylus spp., Dictyocaulus spp., Muellerius spp., Protostrongylus spp., Neostrongylus spp., Cystocaulus spp., Pneumostrongylus spp., Spicocaulus spp., Elaphostrongylus spp. Parelaphostrongylus spp., Crenosoma spp., Paracrenosoma spp., Angiostrongylus spp., Aelurostrongylus spp., Filaroides spp., Parafilaroides spp., Trichostrongylus spp., Haemonchus spp., Ostertagia spp., Marshallagia spp., Cooperia spp., Nematodirus spp., Hyostrongylus spp., Obeliscoides spp., Amidostomum spp., Ollulanus spp.

Aus der Ordnung der Spirurida z. B.: Oxyuris spp., Enterobius spp., Passalurus spp., Syphacia spp., Aspiculuris spp., Heterakis spp.; Ascaris spp., Toxascaris spp., Toxocara spp., Baylisascaris spp., Parascaris spp., Anisakis spp., Ascaridia spp.; Gnathostoma spp., Physaloptera spp., Thelazia spp., Gongylonema spp., Habronema spp., Parabronema spp., Draschia spp., Dracunculus spp.; Stephanofilaria spp., Parafilaria spp., Setaria spp., Loa spp., Dirofilaria spp., Litomosoides spp., Brugia spp., Wuchereria spp., Onchocerca spp.

**Acantocephala:** Aus der Ordnung der Oligacanthorhynchida z. B: Macracanthorhynchus spp., Prosthenorchis spp.; aus der Ordnung der Polymorphida z. B.: Filicollis spp.; aus der Ordnung der Moniliformida z.B.: Moniliformis spp.,

Aus der Ordnung der Echinorhynchida z. B. Acanthocephalus spp., Echinorhynchus spp., Leptorhynchoides spp.

**Pentastoma:** Aus der Ordnung der Porocephalida z. B. Linguatula spp.

Gemäß einer bevorzugten Ausführungsform werden die erfindungsgemäßen Zubereitungen bzw. die erfindungsgemäßen Arzneimittel zur Bekämpfung des Herzwurms Dirofilaria immitis eingesetzt.

Tiere können Fische, Reptilien, Vögel oder insbesondere Säugetiere sein.

Zu den Nutz- und Zuchttieren gehören Säugetiere wie z. B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten, Strauße, Fische wie Forellen, Lachse, Karpfen, Barsche, Hechte, Aale.

Zu Labor- und Versuchstieren gehören Mäuse, Ratten, Meerschweinchen, Goldhamster, Hunde und Katzen.

Zu den Hobbytieren gehören Hunde und Katzen.

Erfindungsgemäß bevorzugt ist die Anwendung bei Tieren, aber grundsätzlich kommt auch die Anwendung am Menschen in Frage.

Die Anwendung kann sowohl prophylaktisch als auch therapeutisch erfolgen.

Die erfindungsgemäßen Zubereitungen mit amorphem Emodepsid zeigen gute Bioverfügbarkeit. Sie weisen eine hohe Plasmaspiegelkonzentration und gute Werte bezüglich der Fläche unter der Konzentrations-Zeit-Kurve von Emodepsid im Blut auf.

### Beispiele

### 1. Copräzipitat hergestellt durch Lösungsmittelverfahren

Bei der Herstellung eines Lösungsmittelcopräzipitates werden Emodepsid und Polyvinylpyrrolidon mit einem K-Wert von 12, 17 oder 25 gemischt und Ethanol oder in einem Lösungsmittelgemisch aus Aceton und Isopropanol gelöst. Nachdem sich alles gelöst hat, wird die Lösung auf ein Blech überführt und das Lösungsmittel im Vakuumtrockenschrank bei erhöhter Temperatur und vermindertem Druck abgezogen. Anschließend wird das so erhaltene Copräzipitat vom Blech abgekratzt und gemahlen. Das so erhaltene Pulver kann nun entweder direkt, z. B. in Kapseln abgefüllt, oder nach Weiterverarbeitung in Form von Tabletten verabreicht werden.

Es wurden Copräzipitate der folgenden Zusammensetzungen hergestellt, wobei die Zusammensetzungen jeweils auf ein theoretisches Gesamtgewicht von 100 g des Copräzipitates umgerechnet wurden. Pro Beispiel wurden jeweils drei Zusammensetzungen mit Polyvinylpyrrolidon-12, -17 bzw. -25 hergestellt:
Beispiel 1:
   25 g Emodepsid
   75 g Polyvinylpyrrolidon-12, -17 oder 25
   Isopropanol/Aceton 1:1 (bis sich alles gelöst hat)
Beispiel 2:
   50 g Emodepsid
   50 g Polyvinylpyrrolidon-12, -17 oder 25
   Isopropanol/Aceton 1:1 (bis sich alles gelöst hat)
Beispiel 3:
   9.09 g Emodepsid
   90,91 g Polyvinylpyrrolidon-12, -17 oder 25
   Isopropanol/Aceton 1:1 (bis sich alles gelöst hat)
Beispiel 4:
   25 g Emodepsid
   75 g Polyvinylpyrrolidon-12, -17 oder 25
   Ethanol (bis sich alles gelöst hat)
Beispiel 5:
   33,33 g Emodepsid
   66,67 g Polyvinylpyrrolidon-12, -17 oder 25
   Ethanol (bis sich alles gelöst hat)
Beispiel 6:
   9.09 g Emodepsid
   90,91 g Polyvinylpyrrolidon-12, -17 oder 25
   Ethanol (bis sich alles gelöst hat)

### 2. Tablettenformulierung:

Bei der Weiterverarbeitung zu Tabletten wird das Copräzipitat mit den Tablettierhilfsstoffen: mikrokristalline Cellulose, Croscarmellose Natrium, hochdisperses Siliciumdioxid, Natriumdodecylsulphat und Magnesiumstearat gemischt und zu Tabletten verpresst.

Als Beispiel sei die folgende Tablettenformulierung genannt:
Beispiel 7: (ein 100 g Ansatz setzt sich zusammen aus)
21,3 g Lösungsmittelcopräzipitat gemäß Bsp. 1 mit Polyvinylpyrrolidon-12
32 g mikrokristalline Cellulose
42,6 g Crosscarmellose Natrium
0,8 g Natriumdodecylsulphat
1,6 g hochdisperses Siliciumdioxid
1,6 g Magnesiumstearat.

### 3. Copräzipitat hergestellt durch Schmelzextrusion

Bei der Zubereitung, welche mittels Schmelzextrusion hergestellt wird (extrudiertes Copräzipitat), werden Emodepsid und das Polyvinylpyrrolidon-Copolymer (Copovidon, z. B. Kollidon VA 64 der Fa. BASF) gemischt und in den Extruder überführt. Bei Bedarf kann ein Tensid, wie z.B. Polyoxyethylenglycerolricinoleat 35, über eine Flüssigdosierung zugeführt werden.Diese Mischung wird bei 160 °C extrudiert. Auch eine Extrusion bei 180 °C ist möglich. Die erhaltenen Emodepsid-Polyvinylpyrrolidonstränge werden abgekühlt und gemahlen. Auch hier kann das Pulver entweder als solches verabreicht oder zu Tabletten weiterverarbeitet werden.

Es wurden extrudierte Copräzipitate der folgenden Zusammensetzungen hergestellt, wobei die Zusammensetzungen jeweils auf ein theoretisches Gesamtgewicht von 100 g des extrudierten Copräzipitates umgerechnet wurden:
Beispiel 8:
   20 g Emodepsid
   70 g Copovidon
   10 g Polyoxyethylenglycerolricinoleat 35
Beispiel 9:
   9,09 g Emodepsid
   80,91 g Copovidon
   10 g Polyoxyethylenglycerolricinoleat 35
Beispiel 10:
   20 g Emodepsid
   80 g Copovidon

### 4. Tablettenformulierung

Auch die Zubereitung, welche durch Schmelzextrusion erhalten wird, lässt sich in gleicher Weise zu Tabletten verarbeiten. Als Beispiel sei die folgende Tablettenmischung genannt:
Beispiel 11 (ein 100 g Ansatz setzt sich zusammen aus):
25,3 g extrudiertes Copräzipitat gemäß Beispiel 8
30,4 g mikrokristalliner Cellulose
40,5 g Crosscarmellose Natrium
0,8 g Natriumdodecylsulphat,
1,5 g hochdisperses Siliciumdioxid
1,5 g Magnesiumstearat.

### Biologisches Beispiel

### A. Studie zur Pharmakokinetik:

Die Tabletten aus Beispiel 7 (mit 10 mg Emodepsid und einem Gesamtgewicht von 187,5 mg) wurden 10 Hunden und die Tabletten aus Beispiel 11 (mit 10 mg Emodepsid und einem Gesamtgewicht von 197,5 mg) 4 Hunden jeweils oral verabreicht. Zum Vergleich wurde eine Emodepsid-Solketallösung (10 % m/m) jeweils 4 Hunden oral verabreicht Die Dosierung betrug bei allen Formulierungen 1 mg/kg Körpergewicht. Anschließend wurde den Hunden in regelmäßigen Abständen, bis zu 72 h nach Applikation, Blut abgenommen. Die Werte der maximalen Plasmaspiegelkonzentration Cmax konnten durch Verwenden des Wirkstoffs in amorphem Zustand deutlich verbessert werden: von 93 µg/L bei der Emodepsid-Solketal-Lösung auf 187 µg/L bei Tabletten aus Beispiel 11 und 246 µg/L bei Tabletten aus Beispiel 7. Die AUC(0-24 h)-Werte betrugen anstatt 508 µg/L bei der Lösung 825 µg/L bei den Tabletten aus Beispiel 11 bzw. 1129 µg/L bei den Tabletten aus Beispiel 7.

## Patentansprüche

1. Zubereitung enthaltend Emodepsid in amorpher Form in einer Polyvinylpyrrolidon-Matrix.

2. Zubereitung gemäß Anspruch 1, **dadurch gekennzeichnet, dass** ein Anteil von mindestens 50 Gew.-% des Emodepsids in amorphen Zustand vorliegt, wobei die Prozentangabe auf die Gesamtmenge an Emodepsid bezogen ist.

3. Zubereitung gemäß Anspruch 1, **dadurch gekennzeichnet, dass** die Polyvinylpyrrolidon-Matrix Polyvinylpyrrolidon oder deren Derivate enthält, die einen K-Wert von 12 bis 30 aufweist.

4. Zubereitung gemäß Anspruch 1, **dadurch gekennzeichnet, dass** das Polyvinylpyrrolidon-Derivat Copovidon ist.

5. Zubereitung gemäß Anspruch 4, **dadurch gekennzeichnet, dass** das Copovidon ein Copolymer aus Vinylpyrrolidon und Vinylacetat im Verhältnis 6:4 mit einem K-Wert von etwa 30 ist.

6. Zubereitung gemäß Anspruch 1, enthaltend zusätzlich ein Tensid.

7. Zubereitung gemäß Anspruch 1 **dadurch gekennzeichnet, dass** sie ein Schmelzextrudat ist.

8. Zubereitung gemäß Anspruch 1 **dadurch gekennzeichnet, dass** sie ein Copräzipitat ist.

9. Arzneimittel enthaltend eine Zubereitung gemäß einem der vorstehenden Ansprüche sowie pharmazeutisch verträgliche Hilfsstoffe.

10. Zubereitung gemäß einem der Ansprüche 1 bis 8 zur Verwendung zur Bekämpfung von Endoparasiten bei Menschen oder Tieren.

11. Zubereitung gemäß Anspruch 10, **dadurch gekennzeichnet, dass** die Endoparasiten Helminthe aus der Gruppe der Nematoden sind.

12. Zubereitung gemäß Anspruch 11, **dadurch gekennzeichnet, dass** die Endoparasiten Nematoden ausgewählt aus der Gruppe der Ordnung der Trichinellida, Tylenchida, Rhabditina, Spirurida sind.

13. Zubereitung gemäß Anspruch 10 oder 11, **dadurch gekennzeichnet, dass** die Nematoden ausgewählt sind aus der Gruppe der Trichuris spp., Trichinella spp., Strongyloides spp., Ancylostoma spp., Enterobius spp., Ascaris spp., Toxascaris spp., Dracunculus spp., Loa spp., Brugia spp., Wuchereria spp., Onchocerca spp.

14. Zubereitung gemäß Anspruch 10 zur Verwendung zur Bekämpfung von Dirofilaria immitis.

15. Verwendung einer Zubereitung gemäß einem der Ansprüche 1 bis 8 zur Herstellung eines Arzneimittels.

16. Verwendung gemäß Anspruch 15 zur Herstellung eines Arzneimittels zur Bekämpfung von Endoparasiten bei Menschen oder Tieren.

17. Verwendung gemäß Anspruch 16 wobei die Endoparasiten Dirofilaria immitis sind.

## Claims

1. Preparation comprising emodepside in amorphous form in a polyvinylpyrrolidone matrix.

2. Preparation according to Claim 1, **characterized in that** a proportion of at least 50% by weight of the emodepside is present in the amorphous state, the percentage being based on the total amount of emodepside.

3. Preparation according to Claim 1, **characterized in that** the polyvinylpyrrolidone matrix comprises polyvinyl pyrrolidone or its derivatives, having a K value of from 12 to 30.

4. Preparation according to Claim 1, **characterized in that** the polyvinylpyrrolidone derivative is copovidone.

5. Preparation according to Claim 4, **characterized in that** the copovidone is a vinylpyrrolidone/vinyl acetate copolymer in the ratio of 6:4 with a K value of approximately 30.

6. Preparation according to Claim 1, additionally comprising a surfactant.

7. Preparation according to Claim 1, **characterized in that** it is a melt extrudate.

8. Preparation according to Claim 1, **characterized in that** it is a coprecipitate.

9. Pharmaceutical, comprising a preparation according to one of the preceding claims and pharmaceutically acceptable adjuvants.

10. Preparation according to one of Claims 1 to 8 for use in the control of endoparasites in humans or animals.

11. Preparation according to Claim 10, **characterized in that** the endoparasites are helminths from the group Nematoda.

12. Preparation according to Claim 11, **characterized in that** the endoparasites are Nematoda from the group of the order Trichinellida, Tylenchida, Rhabditina, Spirurida.

13. Preparation according to Claim 10 or 11, **characterized in that** the Nematoda are selected from the group of Trichuris spp., Trichinella spp., Strongyloides spp., Ancylostoma spp., Enterobius spp., Ascaris spp., Toxascaris spp., Dracunculus spp., Loa spp., Brugia spp., Wuchereria spp., Onchocerca spp.

14. Preparation according to Claim 10 for use in the control of Dirofilaria immitis.

15. Use of a preparation according to one of Claims 1 to 8 for the preparation of a pharmaceutical.

16. Use according to Claim 15 for the preparation of a pharmaceutical for controlling endoparasites in humans or animals.

17. Use according to Claim 16, where the endoparasites are Dirofilaria immitis.

## Revendications

1. Préparation contenant de l'émodepside sous forme amorphe dans une matrice de polyvinylpyrrolidone.

2. Préparation selon la revendication 1, **caractérisée en ce qu'**une proportion d'au moins 50 % en poids de l'émodepside se présente à l'état amorphe, l'indication de pourcentage se rapportant à la quantité totale d'émodepside.

3. Préparation selon la revendication 1, **caractérisée en ce que** la matrice de polyvinylpyrrolidone contient de la polyvinylpyrrolidone ou ses dérivés, qui présentent une valeur K de 12 à 30.

4. Préparation selon la revendication 1, **caractérisée en ce que** le dérivé de polyvinylpyrrolidone est la copovidone.

5. Préparation selon la revendication 4, **caractérisée en ce que** la copovidone est un copolymère de vinylpyrrolidone et d'acétate de vinyle en un rapport de 6:4 ayant une valeur K d'environ 30.

6. Préparation selon la revendication 1, contenant en outre un tensioactif.

7. Préparation selon la revendication 1, **caractérisée en ce qu'**elle consiste en un extrudat de masse fondue.

8. Préparation selon la revendication 1, **caractérisée en ce qu'**elle consiste en un coprécipité.

9. Médicament contenant une préparation selon l'une quelconque des revendications précédentes, ainsi que des adjuvants pharmaceutiquement compatibles.

10. Préparation selon l'une quelconque des revendications 1 à 8, destinée à une utilisation pour lutter contre les endoparasites chez les hommes ou les animaux.

11. Préparation selon la revendication 10, **caractérisée en ce que** les endoparasites sont des helminthes du groupe des nématodes.

12. Préparation selon la revendication 11, **caractérisée en ce que** les endoparasites sont des nématodes choisis dans le groupe des ordres des Trichinellida, Tylenchida, Rhabditina, Spirurida.

13. Préparation selon la revendication 10 ou 11, **caractérisée en ce que** les nématodes sont choisis dans le groupe constitué par Trichuris spp., Trichinella spp., Strongyloides spp., Ancylostoma spp., Enterobius spp., Ascaris spp., Toxascaris spp., Dracunculus spp., Loa spp., Brugia spp., Wuchereria spp., Onchocerca spp.

14. Préparation selon la revendication 10, destinée à une utilisation pour lutter contre Dirofilaria immitis.

15. Utilisation d'une préparation selon l'une quelconque des revendications 1 à 8 pour la fabrication d'un médicament.

16. Utilisation selon la revendication 15 pour la fabrication d'un médicament pour lutter contre des endoparasites chez les hommes ou les animaux.

17. Utilisation selon la revendication 16, dans laquelle les endoparasites sont Dirofilaria immitis.
